# EUROPEAN PATENT SPECIFICATION

(11) **EP 0 799 821 B1**
(45) Date of publication and mention of the grant of the patent: **13.03.2002**
(21) Application number: 97105611.4
(22) Date of filing: 04.04.1997
(51) Int. Cl.: C07C 237/10, C07C 237/06, C08G 18/18

(54) **Process for the synthesis of polyurethane using reactive amine catalysts**
Verfahren zur Synthese von Polyurethanen mit reaktiven Aminkatalysatoren
Procédé de synthèse de polyuréthanes utilisant des catalyseurs aminés

(30) Priority: 04.04.1996 US 14843
(43) Date of publication of application: 08.10.1997
(73) Proprietor: CROMPTON CORPORATION, Greenwich Connecticut 06831-2559 (US)
(72) Inventor: Gerkin, Richard M., Cross Lanes, W.Va. 25313 (US); Robinson, Kaye K., Scott Depot, W.Va. 25560 (US)
(74) Representative: Spott, Gottfried, Dr.

(56) References cited:
- US-A- 4 105 600
- US-A- 4 143 071
- US-A- 4 256 665
- CHEMICAL ABSTRACTS, vol. 118, no. 14, 5 April 1993 Columbus, Ohio, US; abstract no. 125107b, K.V.SHIRSHIN ET AL.: "Interaction of methyl (meth)acrylate with N,N-dimethylaminopropylamine" XP002035572 & ZH. PRIKL. KHIM., vol. 65, no. 6, pages 1379-83,

## Description

Certain amine catalysts are known in the polyurethane industry such as propanamide, N,N-dimethyl-3-[dimethylamino] (DDPA, Structure 1), which is the simplest of a series of catalysts, having no reactive functional groups, for the formation of polyurethane described in U.S. Patent No. 4,011,223. 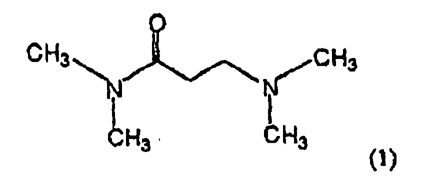 A similar non-reactive analog that has been found useful as a polyurethane catalyst is propanamide, 3-[bis-(3-(dimethylamino]propyl)]amino-N,N-dimethyl as described in U.S. Patent No. 4.049,591.

Additionally, a number of hydroxyl, and primary/secondary amine containing tertiary amine polyurethane catalysts are described in the article "Factors Affecting the Discoloration of Vinyl That Has Been Molded Against Urethane Foam," R. L. Zimmerman and T. L. Austin, Polyurethane World Congress 1987, Sept. 29-Oct. 2, pp. 693-697, 1987. However, all of these catalysts have deficiencies in either activity, with the hydroxy substituted cases, or with volatility, such as in the unsubstituted case.

U.S. Patent No. 4,384,950 describes the use of a substituted form of DDPA as a demulsifier for breaking oil-in-water emulsions from tar-sand bitumen recovery. The reference, however, does not describe the use of this compound as a catalyst for urethane systems. The reaction used in the preparation of the substituted compound involves addition/condensation of methacrylic or acrylic acid with dimethylaminopropylamine. Methods of manufacture of said compound are disclosed in U.S. Patent Nos. 4,256,665 and 4,259,259.

### Summary of the Invention

The present invention provides amine/amide catalysts for use in catalyzing the formation polyurethane. The amine/amide catalysts have low volatility in the resulting polyurethane (i.e., low fogging) and reactivity at least as good as the most reactive component in the system [see Priester, R. D. Jr., R. D. Pefley and R. B. Turner, "High Resiliency Polyurea Foam - An Improved Flexible Foam Matrix, Journal of Cellular Plastics, 30(2) 1994, pp. 147, which is incorporated herein by reference]. These compounds are tertiary amine/amides that have similar base structures to DDPA, but contain secondary amine groups for reacting into the polymer matrix. Unexpectedly, these highly reactive compounds have a catalytic activity which is very close to that of the completely unsubstituted catalyst DDPA.

The structure of the tertiary amine/amides of the present invention is: 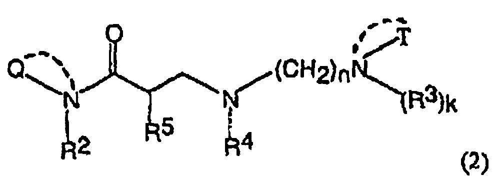 Q is C_{z}H_{2z+1}, or (CH₂)ₙN(R³)ₖT and T is a divalent cyclic group, by which is meant a group which is attached at both its ends to the nitrogen to form a cyclic group, or T is monovalent alkyl, aminoalkyl or alkylaminoalkyl group, k = 0 or 1, being 1 if T is a monovalent group and 0 if T is a divalent group; R² = H or C_{z}H_{2z+1}; each occurrence of R³ = C_{z}H_{2z+1}; R⁴= H; R⁵ = H or CH_{3:n} = 2 to 6; and each occurrence of z = 1 to 4. "n" is preferably 2 or 3 and z is preferably 1. T when monovalent may be an alkyl group of one to four carbons which may have one or more amines thereon (e.g., amino-C₁-C₄- alkyl) or therein (e.g., mono- or di-C₁-C₄-alkylamino-C₁-C₄- alkyl), T when divalent may be alkyl, amine substituted alkyl, alkylaminoalkyl, or alkoxyalkyl which forms with the nitrogen atom shown in structure (2) to which T is attached a cyclic structure which incorporates up to 6 carbon atoms in the ring as well as the nitrogen atom shown in structure (2) and optionally a second nitrogen atom or an oxygen atom in the ring, e.g., morpholino, piperazino. Said cyclic structures may include C₁ to C₄ alkyl substitutions on the ring.

The present invention provides methods of forming polyurethane by combining the polyol and polyisocyanate reactants in the presence of an effective amount of one or more than one compound of formula (2) to catalyze the reaction of said reactants.

### Detailed Description of the Invention

A preferred subset of the amine/amides of the present invention is: 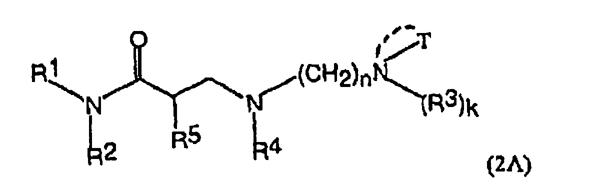 or more particularly 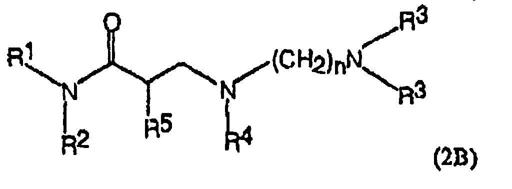 wherein R¹ = (R³)₂N(CH₂)ₙ or C_{z}H_{2z+1}; R², R³, R⁴, R⁵, T, k, n and z are as above, "n" is preferably 2 or 3 and z is preferably 1. Each R³ may be the same or different, as may each value of n and z. One specific preferred range of structures for 2A and 2B is those in which R¹ is C_{z}H_{2z+1}. Preferred specific compounds are: 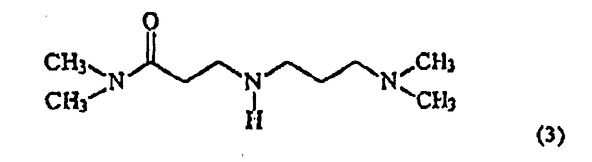 and

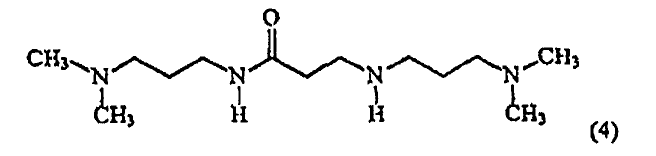

An example of such a cyclic terminated structures is:

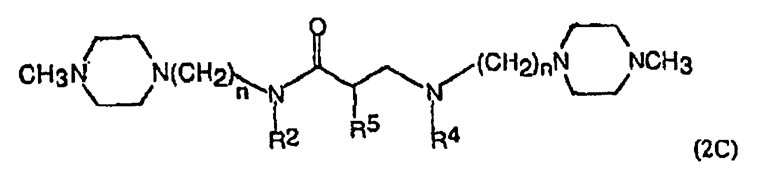

These compounds may be manufactured as known in the art for the manufacture of amine/amide. Generally, the catalysts are prepared from the direct reaction of dimethylaminopropylamine (DMAPA) or other similar amines, with methyl acrylate (MA), dimethyl acrylamide (DMAA) or similar unsaturated materials. The products of these reactions are substantially the aminopropionamides of the present invention containing lesser amounts of unreacted raw materials and other adducts such as: 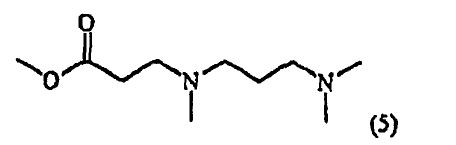 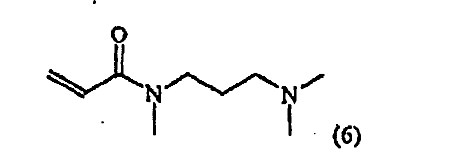 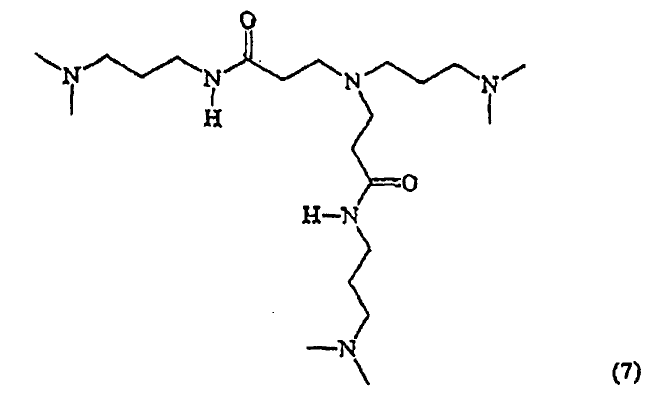 Methods of manufacture of compounds of Structure 4 are specifically disclosed in U.S. Patent Nos. 4,256,665 and 4,259,259.

These amine/amide catalysts are used for the catalysis of the reaction to form polyurethane, i.e., catalyze the isocyanate/water and/or isocyanate/alcohol reactions. Said polyurethanes may be rigid, flexible slabslock, ester slabstock, molded microcellular elastomer or other types of foams as are known in the art. The amine/amides of the present invention can be used in amine pre-blends, i.e., mixtures with other amine catalysts, surfactants, or other additives or polyurethane components as are known in the art.

Foam formulations with which the compounds of the present invention can be used as catalysts usually comprise (a) a polyether polyol containing an average of more than two hydroxyl groups per molecule; (b) an organic polyisocyanate; (c) at least one catalyst for production of polyurethane foam; (d) water; (e) a surfactant, preferably any of the silicone/polyether copolymers known in this field for this purpose; and (f) an inert gas.

The polyols have an average number of hydroxyl groups per molecule of at least slightly above 2 and typically 2.1 to 3.5. Generally, the polyol should have an equivalent weight of 400 to 1500 or even 400 to 3000 grams/equivalent and an ethylene oxide content of less than 20%. Useful polyols include but are not limited to polyether polyols such as alkylene oxide adducts of polyhydroxyalkanes, alkylene oxide adducts of non-reducing sugars and sugar derivatives, alkylene oxide adducts of polyphenols, and alkylene oxide adducts of polyamines and polyhydroxyamines. The alkylene oxides are preferably based on ethylene oxide or propylene oxide.

The organic polyisocyanates contain at least two isocyanate groups, e.g., toluene diisocyanates (TDI), and the index of the foam is typically 60 to 130.

The water generally comprises on the order of 1 to 12 php (parts by weight per hundred parts of polyol).

Other additives may be added to the polyurethane foam to impart specific properties to the foam, including, but not limited to, coloring agents, flame retardants, and GEOLITE® Modifier foam additives (available from Organo Silicones Group of Witco Corporation, Greenwich, CT). The inert gas is one which is soluble in the foam formulation at elevated pressures, but will come out of solution (i.e., blow) at atmospheric pressure. An exemplary such gas is CO₂, but nitrogen, air or other common gases, including hydrocarbon gases, such as methane and ethane may also be used. The inert gas may also comprise a volatile organic compound such as a pentane isomer or a hydrochlorocarbon that boils above ambient temperature but has a sufficiently high vapor pressure at ambient temperature that its vapor represents a substantial component of the gas in the cells of the foam.

The silicone copolymer surfactants should be capable of helping to form a stable foam and should be present in an amount effective to stabilize the polyurethane foam, i.e., an amount which is generally 0.05 to 5 wt. percent of the total reaction mixture, preferably 0.2 to 1.5 wt. percent.

The foam is manufactured by mixing the ingredients (that is, ingredients (a) through (f) together such that by product gas generated during the reaction foams the polyurethane. The foam can also be made by the injection of inert gas, whereby the reactants are put under high pressure (i.e., at least greater than atmospheric pressures) so that the inert gas is dissolved in the reactant mixture. Then the mixture is flashed, by releasing the pressure, which causes the gas to form bubbles at nucleation sites in the foaming system and thus act as a blowing agent. This produces a reduced density foam. For a more complete description of this process and the equipment required therein, see European Patent Publication No. 0 645 226 A2, which is incorporated herein by reference.

The compounds of the present invention may also be used in non-foam polyurethane reactions, such as polyurethane elastomer formation. In such polyurethanes, the water in the formulation is often replaced with a chain extender, which is a low molecular weight (<400) active hydrogen containing compound with at least two reactive groups. Examples are 1,4-butanediol, ethylene glycol, diethylene glycol and ethylene diamine.

The conditions and formulations for these reactions are known in the art, e.g., "Polyurethane Handbook," 2nd ed., Gunter Ortel, ed., Hanser Publishers, Cincinnati, 1994, which is incorporated herein by reference. Generally, these catalysts are used at a catalytically effective amount, i.e., in an amount to effectively catalyze the reaction to form the polyurethane. Generally said effective amount is 0.02 - 5.0 parts per hundred parts of polyol in the reaction formulation. In molded flexible foam, which is described in the examples below, these catalysts resulted in cream and exit times slightly faster than for DDPA, and the load properties (ILD) and cure characteristics of the foams were at least as good as for DDPA.

### EXAMPLES

### Glossary:

- php:: Parts of product per 100 parts of polyol in the formulation.
- Polyol 1:: An ethylene oxide/propylene oxide polyether sold by ARCO Chemical as ARCOL Polyol E-656.
- Polyol 2:: An ethylene oxide/propylene oxide polyether sold by ARCO Chemical as ARCOL Polyol E-688.
- Polyol 3:: A propylene oxide polyether sold by Dow Chemical as VORANOL 490.
- Polyol 4:: A propylene oxide polyether sold by Dow Chemical as VORANOL 800.
- Polyol 5:: A polyester polyol sold by Stepan Chemical as PS-3152.
- Polyol 6:: A polyester polyol sold by Witco as FOMREZ 53.
- Silicone 1:: A silicone surfactant sold by Witco as NIAX surfactant L-3001.
- Silicone 2:: A silicone surfactant sold by Witco as NIAX surfactant X-10829.
- Silicone 3:: A silicone surfactant sold by Witco as NIAX surfactant L-6900.
- Silicons 4:: A silicone surfactant sold by Witco as L-532.
- Surfactant 1:: An organic surfactant sold by Union Carbide Corp. as NP-9.
- Catalyst 1:: An amine catalyst sold by Witco as NIAX catalyst A-1.
- Catalyst 2:: An amine catalyst sold by Witco as NIAX catalyst A-33.
- Catalyst 3:: An amine catalyst sold by Witco as NIAX catalyst A-99.
- Isocyanate 1:: A diphenyl methylene diisocyanate (MDI) variant sold by Dow Chemical as ISONATE 143-L
- Isocyanate 2:: The standard commercial mixture of 80% 2,4 and 20% 2,6 toluene diisocyanate.
- Isocyanate 3:: An MDI variant sold commercially by Dow Chemical as PAPI 27.
- IFD:: Foam load values as determined by ASTM D-3574 Test Bi

### General Synthesis: Uncatalyzed reaction of certain primary amine containing tertiary amines with acrylates or methacrylates

The synthesis of the following tertiary amine/amides was conducted in a 500 mL roundbottom four-neck flask. The flask was equipped with a pressure equalizing addition funnel, mechanical stirrer, nitrogen purge, thermometer and heating mantle. Either one mole of DMAA and one mole of the amino of interest, or one mole of MA and two moles of the amine were used. If the amine it was primary, it was weighed into the flask and the DMAA or MA was weighed into the addition funnel. If the amine was not primary, the order was reversed (i.e., the amine was placed in the addition funnel and the DMAA or MA in the flask). Specific details of reactions are outlined below.

### Example 1 - Synthesis of Amines/Amides of the Present Invention

Propanamide 3-[3-dimethylaminopropyl]amino-N,N-dimethyl- One mole of the DMAPA (dimethylaminopropylamine, 102.21 g) was weighed into the flask. The system was purged with nitrogen for several minutes. The DMAA was added (6 mL/min)to the flask while the mixture was being stirred and the temperature was being monitored. The initial temperature was 24° C and did not change during the addition. Once the DMAA addition was complete, the flask was heated to 100°C and held for two hours with stirring. Structure #3 above was obtained at 90+% conversion.

Propanamide 3-[3-dimethylaminopropyl]amino-N-[3-dimethylaminopropyl]- The synthesis of the MA/DMAPA version of the amine was conducted by the procedure above using two moles of DMAPA (204.42 g) and one mole of MA (86.10 g). During the addition of the MA the temperature increased from and initial temperature at 24°C to a final temperature 75°C. The temperature was held at 75°C for two hours. The sample was then stripped on a rotary evaporator for four hours at 70°C, 5 mm Hg to remove methanol. Structure #4 above was obtained at 92+% conversion.

Propanamide 3-[3-dimethylaminopropyl]amino-N-[3-dimethylaminopropyl],2-methyl- The synthesis of the methylmethacrylate (MMA)/DMAPA version of the amine was conducted by the procedure listed above using two moles of DMAPA (204.42 g) and one mole of mMA (86.10 g). During the addition of the MMA the temperature did not change from the initial temperature of 24° C. The temperature was increased to 120°C for a total of twenty-four hours. The sample was stripped on a rotary evaporator for four hours at 70°C, 5 mm Hg to remove methanol. The following structure was obtained at approximately 80% conversion:

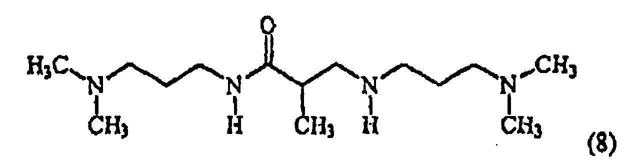

### Example 2 - Synthesis of Comparative Catalysts

Propanamide, 3-[dimethyl]amino-N,N-dimethyl was made, starting from DMAA (one mole) was added to a stirred reactor under nitrogen. Dimethylamine (one mole) was added at such a rate as to keep the temperature in the reactor <35°C. When all of the dimethylamine was added, the reactor was held between 35 and 45°C for about two hours. After that time, temperature was increased to 60°C for an additional 5 hours. After cooling to 25°C, the reaction was complete and the product analyzed. The analysis confirmed the anticipated structure of DDPA at 99+% conversion. Similarly, propanamide, 3-[3-methyl-3-hydroxyethyl]amino-N,N-dimethyl (9) was made from DMAA and MEOA (N-methylethanolamine). 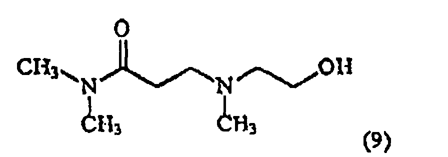 Additionally, propanamide, 3-[bis(2-hydroxyethyl)amino]-N,N-dimethyl (10) was made from DMAA and DEOA (diethanolamine). 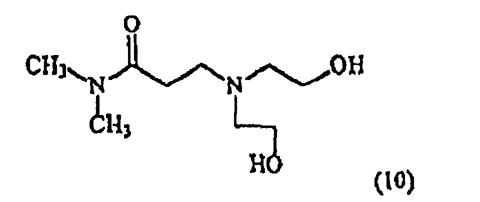 Propanamide, 3-[3-methyl-3-hydroxyethyl]amino-N-methyl-N-hydroxyethyl (11) was made from MA and MEOA. 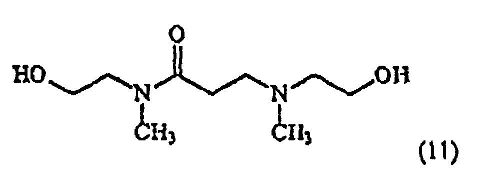 Propanamide, 3-[bis(2-hydroxyethyl)amino]-N,N-[bis(2-hydroxyethyl)] (12) was made from MA and DEOA.

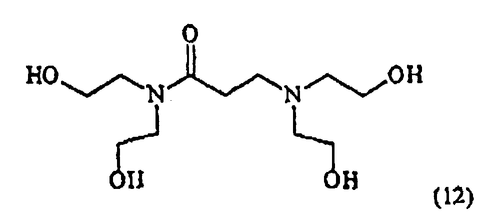

### Example 3 - Evaluation in a Simple Water Blown Urethane Foam

Each of the reactive DDPA catalysts was evaluated in terms of its blow and gel capabilities relative to DDPA. To obtain blow capabilities a simple system of 97.22 php (0.049 eq.) of Polyol 1, 1.79 php (0.195 eq.) of water. 1 php of Surfactant 1 and Isocyanate 1 at 103 index was used. A total of 50 grams of premade polyol, water, surfactant blend was weighed into a lined one pint paper cup. The catalysts were evaluated by adding 0.25g (0.5 php) or 0.5g (1.0 php) to this mixture. Isocyanate was added and the mixture stirred on a drill press for 5 seconds. Blow capabilities were determined by measuring top-of-cup and blow-off times as compared to DDPA. Data are presented in Table 1.

**Table 1 -**

| **Water Blown Foam Examples** | | | | | |
|---|---|---|---|---|---|
| **Run** | **Structure #** | **php** | **Top-of-Cup (sec)**^{**a**} | **Krel DDPA**^{**b**} | **Blow (s)**^{**c**} |
| 1 | 1 | 0.5 | 168 | 1.00 | >300 |
| 2 | 1 | 1.0 | 53 | 1.00 | 114 |
| 3 | 4 | 0.5 | 157 | 0.93 | 300 |
| 4 | 4 | 1.0 | 60 | 1.13 | 113 |
| 5 | 3 | 0.5 | 190 | 1.13 | >300 |
| 6 | 3 | 1.0 | 130 | 2.45 | 235 |
| 7 | 9 | 0.5 | >600 | >3.57 | >600 |
| 8 | 9 | 1.0 | 227 | 4.28 | >300 |
| 9 | 11 | 0.5 | >600 | >3.57 | >600 |
| 10 | 11 | 1.0 | 236 | 4.45 | >300 |
| 11 | 10 | 0.5 | >600 | >3.57 | >600 |
| 12 | 10 | 1.0 | >600 | >11.3 | >600 |
| 13 | 12 | 0.5 | >600 | >3.57 | >600 |
| 14 | 12 | 1.0 | >600 | >11.3 | >600 |

| | | | | | |
|---|---|---|---|---|---|
| ^{a} The top-of-cup time represents the time (seconds) at which the rising form reached height of the cup. | | | | | |
| ^{b} The Krel DDPA value represents the relative activity of the catalyst and was obtained by dividing the top-of-cup time for the amine-amide by the top-of-cup time for the DDPA at a given use level. For Run 3; 157 sec/168 sec = 0.93 = Krel DDPA at 0.5 php. | | | | | |
| ^{c} The blow time represents the time (seconds) at which gasses visibly escaped from the foam. | | | | | |

Table 1 shows this comparison in a simple water blown urethane foam formulation. Each catalyst was evaluated at levels of 0.5 and 1.0 php, and rise (top-of cup) and blow-off times noted. It is clear that the candidates break down into two families, those with activities reasonably close to that of the control DDPA (Runs 2-6) and those with significantly poorer activity (Runs 7-14). The foams of Runs 3-6 contain the preferred catalysts noted above (Structures 3 and 4), white Runs 7-14 were catalyzed with the poorer performing hydroxyl containing candidates (Structures 9-12). The difference in overall performance is significant, suggesting unique catalytic behavior of the preferred structures.

### Example 4 - Evaluation in a Urethane Elastomer

A similar experiment was used to evaluate the gel capabilities of the reactive DDPA catalysts relative to DDPA. The resin blend consisted of 94 php (0.047 eq.) of Polyol 1 and 6 php (0.193 eq.) of ethylene glycol. Isocyanate 1 was used at 103 index. A total of 100 grams of the resin blend was weighed into a lined pint-size paper cup. Isocyanate was added and stirred by hand. The catalysts were evaluated at 3 php. Gel capabilities were determined by measuring gel time (point at which mixture was too viscous to stir by hand) and tack-free time as compared to DDPA. Data are presented in Table 2.

**Table 2 -**

| **Urethane Elastomer Examples** | | | | |
|---|---|---|---|---|
| **Run** | **Structure #**^{**a**} | **gel (s)**^{**b**} | **Krel DDPA**^{**c**} | **tack-free (s)**^{**d**} |
| 1 | 1 | 26 | 1.00 | 26 |
| 2 | 4 | 42 | 1.62 | 48 |
| 3 | 3 | 70 | 2.69 | 80 |
| 4 | 9 | 170 | 6.54 | 210 |
| 5 | 11 | 180 | 6.92 | 255 |
| 6 | 10 | >600 | >26 | >600 |
| 7 | 12 | >600 | >26 | >600 |

| | | | | |
|---|---|---|---|---|
| ^{a} All catalysts were evaluated at 3.0 parts, Amine equivalents based only on tertiary amine content @ 3.0 part use level. | | | | |
| ^{b} The gel time represents the time at which the mixture is to viscous to be stirred by hand. | | | | |
| ^{c} The Krel DDPA value represents the relative activity of the corresponding catalyst as compared to DDPA. (gel time of amine-amide of interest/gel time of DDPA). | | | | |
| ^{d} The tack-free time represents the time at which the mixture is tack free to the touch. | | | | |

Runs 2 and 3 confirm that Structures 3 and 4 have activities reasonably close to that of DDPA, while all of the other candidates are very much slower. This significant difference in this elastomer system confirms the unique catalytic character of these compounds and the broad scope of their utility.

### Example 5 - Evaluation in a Molded Flexible Foam Formulation

The catalysts were then evaluated in a molded flexible foam formulation. The control formulation contained 80 php of Polyol 1,20 php of Polyol 2, 1.2 php Silicone 2,1.5 php of DEOA, 3.56 php of water, 0.23 php of Catalyst 2,0.14 php of Catalyst 1, and 0.25 php of "DDPA blend" (see Table 3). Isocyanate 2 was used at an Index of 100. The reactive DDPA catalysts were blended exactly like the "DDPA blend", replacing the DDPA with each catalyst to be evaluated. The new blends were used in place of the "DDPA blend" at equal parts in the formulation. The mixture was mixed (drill press) for 55 seconds, the isocyanate was added and mixed for another 5 seconds after the isocyanate addition. The foams were made in an aluminum mold with 1/16 inch vents. The mold temperature was 150°F (tempered water heating) with a demold time of 3.5 minutes. Foams made were compared by measuring cream and exit times, 50% or 75% ILD values, and cure response. Data are presented in Table 3.

**Table 3 -**

| **Catalyst Comparison in a Flexible Molded Foam** | | | | | | | |
|---|---|---|---|---|---|---|---|
| **Run** | **Structuro #**^{**c**} | **php** | **cream, sec** | **exit time**^{**a**} | **50% ILD**^{**b**} | 75% ILD | cure |
| 1 | 1 | 0.25 | 5 | 40.5 | 152 | 230 | OK |
| 2 | 4 | 0,25 | <5 | 36,9 | nm^{d} | 240 | OK |
| 3 | 4 | 0.50 | 3.5 | 36.4 | nm | 270 | OK |
| 4 | 3 | 0.25 | 5 | 37.8 | nm | 225 | OK |
| 5 | 3 | 0.50 | 3.5 | 36.6 | nm | 220 | OK |
| 6 | 9 | 0.25 | 5 | 42 | 115 | nm | OK |
| 7 | 9 | 0.50 | 5 | 40.8 | 125 | mn | OK |
| 8 | 12 | 0.25 | 5 | 43.5 | nm | 210 | OK |
| 9 | 12 | 0.50 | 5 | 42.8 | nm | 223 | OK |
| 10 | 11 | 0.25 | 5 | 40 | nm | 195 | OK |
| 11 | 11 | 0.50 | 3.5 | 39.8 | nm | 200 | OK |
| 12 | 10 | 0.25 | 5 | 40.5 | nm | 162 | sl set^{e} |
| 13 | 10 | 0.50 | 4 | 39.8 | nm | 174 | OK |

| | | | | | | | |
|---|---|---|---|---|---|---|---|
| ^{a} The exit time represents the time at which the first amount of foam was visible in the mold vents. | | | | | | | |
| ^{b} ILD indicates indentation load deflection. | | | | | | | |
| ^{c} Indicated amine-amide catalyst, 33.5%/ TERGITOL 15.S.7 surfactant (Union Carbide Corp.) 66.5%. | | | | | | | |
| ^{d} nm indicates that this value was not measured. ^{e} sl set indicates that the cure was slow | | | | | | | |

The performance of the preferred catalysts is given in Runs 2-5. These catalysts resulted in cream and exit times slightly faster than those for the DDPA control blend, and the load properties (ILD) and cure characteristics of the foams were at least as good as the control. This is additional evidence that the typical catalytic activity of these tertiary amine/amide compounds is very close to that of DDPA. The performance of the hydroxyl containing candidates is shown in Runs 6-13. While similar to DDPA, they tend to give somewhat longer exit times (i.e., are slower to react) and lower load properties.

### Example 6 - Confirmation of the reactivity of amine/amide with isocyanate

Structure 4 (0.315g. 0.00265m) was added to a small reactor followed by phenyl isocyanate (0.684g, 0.00265m). Immediately upon mixing, there was a significant exotherm and a notable increase in the viscosity of the mixture suggesting a fast reaction. After about three minutes, a sample of the mixture was taken which confirmed that all of the phenyl isocyanate had been consumed. This result confirms that these compounds react readily with isocyanate, supporting the concept that they will react into the foam and be non-volatile.

### Example 7 - Evaluation in Rigid Foam Formulation

The catalysts were evaluated in a rigid foam formulation containing 60 php of Polyol 3,15 php of Polyol 4, 25 php of Polyol 5, 2 php Silicone 3, 1.0 php of water, 36 php of HCFC-141b blowing agent. Isocyanate 3 was used at an Index of 120. Structure 4 was used as the catalyst for evaluation. All components except the isocyanate were premixed in a pint-size lined paper cup. The mixture was mixed (drill press) for 10 seconds, the isocyanate was added and mixed for another 3 seconds. The mixture was then transferred to a lined paper bucket and cream, string and gel, and final rise times were measured. Data represented in Table 4 demonstrate that catalyst 4, of the present invention yielded good rigid foam,

**Table 4 -**

| **Catalyst Evaluation in Rigid Foam** | | | | |
|---|---|---|---|---|
| **grams** | 1.13 | 1.13 | 2.20 | 2.30 |
| **cream (sec)** | 29.23 | 29.65 | 18.60 | 16.14 |
| **string/gel (sec)** | 87.17 | 77.87 | 51.02 | 50.38 |
| **tack-free (set)** | 88.25 | 116.50 | 60.52 | 59.86 |
| **final rise (sec)** | 140.00 | 160.47 | 101.38 | 100.00 |
| **density (pcf)** | 1.85 | 1.85 | 1.81 | 1.82 |

### Example 8 - Evaluation in Polyester Foam Formulation

Structure 4 was also evaluated in a polyester foam formulation. Two foams were made: a control and a foam using structure 4 as a replacement for DDPA at equal amine equivalents. The formulations are listed in Table 5 below. The TDI was added to the polyol and the mixture was hand mixed until it was clear. Then, the polyol/TDI mixture was mixed at 1000 rpm for 8 seconds. The water, amine, surfactant premix was added with a syringe and mixing was continued for 7 seconds. The mixture was then immediately poured into a card box (20 x 20 x 20 cm) and cream and blow times were monitored along with the rise profile. Cream times of the control foam and experimental foam were 13 seconds each. Blow times for the two foams were 119 seconds and 121 seconds, respectively. No differences in the two foams were observed.

**Table 5 -**

| **Catalyst Evaluation in Polyester Foam Formulation** | | |
|---|---|---|
| Components | Control foam, php | Structure 4, php |
| Polyol 6 | 100 | 100 |
| Water | 4 | 4 |
| Silicone 4 | 1.4 | 1.4 |
| Surfactant 1 | 0.42 | 0.43 |
| Catalyst 3 | 0.175 | 0.175 |
| DDPA | 0.105 | -- |
| Structure 4 | -- | 0.095 |
| Isocyanate 2 (103 index) | 48.29 | 48.29 |
| Cream, seconds | 13 | 13 |
| Blow, seconds | 119 | 121 |

### Example 9 - Verification of Nonfugitivity

Fugitivity studies were also performed on a series of polyester foams made using each of the following catalysts: N-ethyl morpholine, N-methyl morpholine, N,N-dimethyl benzylamine, n-hexadecyldimethylamine, and Structure 4. The following formulation was used: Polyol 6, water, silicone 4, surfactant 1, Isocyanate 2 at 103 index. Each of the catalysts was evaluated at the use level required to give a blow time of 41 seconds. The Polyol was weighed into a 32 oz. paper cup. The TDI was added to the polyol and the mixture was hand mixed until it was clear. Then, the polyol/TDI mixture was mixed at 1000 rpm for 8 seconds. The water, amine, surfactant premix was added with a syringe and mixing was continued for 7 seconds. The mixture was then immediately poured into a paper bucket and top-of-cup times were recorded.

Then, approximately 0.2 gram samples of each of the foams were taken from the center of a foam sample cut from the second inch from the bottom of the bucket. These samples were placed in glass vials and sealed and were analyzed on a DB-1 (30 meter x 0.32 mm) column using a Varian 3760 Gas Chromatograph equipped with a Perkim Elmer HS-40 Headspace Autosampler. Data are shown below.

**Table 6 -**

| **Fugitivity Data** | | |
|---|---|---|
| **Catalyst** | **Use Level**^{**a**}**phb** | **Normalized Area Counts** |
| NEM | 2.2 | 179,166,699 |
| NMM | 1.6 | 150,597,591 |
| N,N-dimethylbenzylamine | 1.6 | 244,905,456 |
| n-hexadecyldimelthylamine | 1.4 | 0^{b} |
| Structure 4 | 1.3 | 0^{b} |

| | | |
|---|---|---|
| ^{a} Required php to give a top-of-cup time of 41 sec. | | |
| ^{b} Less than detection limit (<1,000,000). | | |

The above data confirm that Structure 4 has no detectable volatility. Thus, we anticipate that Structure 4 would be nonfugitive in foam applications.

## Claims

1. A process for the formation of polyurethane, comprising reacting a polyol component and a polyisocyanate component in the presence of an effective amount of a catalyst and in the presence of an effective amount of an amine/amide of the structure: 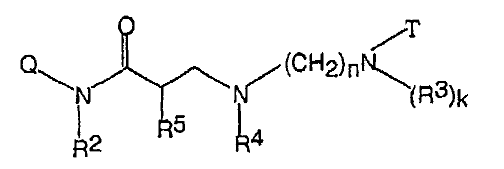 wherein
Q is C_{z}H_{2z+1}, or (CH₂)ₙN(R³)ₖT;
T is a monovalent C₁-C₄ alkyl, amino-C₁-C₄- alkyl, mono-C₁-C₄- alkylamino-C₁-C₄- alkyl, or di-C₁-C₄- alkylamino-C₁-C₄-C₁-C₄- alkyl group, or T is a divalent alkyl, amine substituted alkyl, alkylaminoalkyl, or alkoxyalkyl group which forms with the nitrogen atom shown in structure (I) to which T is attached a cyclic structure which incorporates up to 6 carbon atoms in the ring as well as the nitrogen atom shown in structure (I), which cyclic structure may be substituted with C₁ to C₄ alkyl;
k = 0 or 1, being 1 if T is a monovalent group and 0 if T is a divalent group;
R² = H or C_{z}H_{2z+1}; each occurrence of R³ = C_{z}H_{2z+1}; R⁴ = H; R⁵ = H or CH_{3;} n = 2 to 6; and each occurence of z = 1 to 4.

2. A process according to claim 1 wherein n is 2 to 3 and z is 1.

3. A process according to claims 1 wherein Q = R¹ = C_{z}H_{2z+1}

4. A process according to any one of the claim 1 to 3 wherein R¹ is methyl.

5. A process according to any one of the claims 1 to 4 wherein R³ is methyl.

6. A process according to any one of the claims 1 to 5 wherein R⁵ is hydrogen.

7. A process according to any one of the claims 1 to 6 wherein the amine/amide is of the structure:

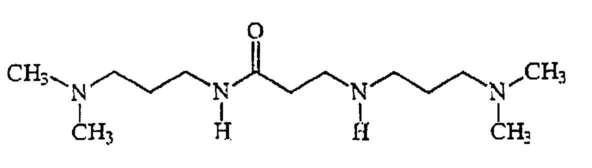

8. A process according to any one of the claims 1 to 6 wherein T is selected from the group consisting of an alkyl of one to four carbon atoms which may have amines thereon or therein or an alkyl, amine substituted alkyl, alkylaminoalkyl, or alkoxyalkyl which forms with the nitrogen atom shown in the formula of claim 1 to which T is attached a cyclic structure which incorporates up to 6 carbon atoms in the ring as well as the nitrogen atom shown in said formula and optionally a second nitrogen atom or an oxygen atom in the ring, wherein said cyclic structure may include C₁ to C₄ alkyl substitutions on the ring.

9. A process according to claim 8 wherein T is an alkyl group of one to four carbons which may have one or more amines thereon.

10. A process according to any one of the claims 1 to 9 wherein the polyol has an equivalent weight of between 400 to 1500 and an average number of hydroxyl groups of 2.1 to 3.5 and the index of the foam is between 60 and 130.

11. Use of an amide/amine of the structure as defined in any one of the claims 1 to 10 as a catalyst for preparing a polyurethane.

## Patentansprüche

1. Verfahren zur Bildung von Polyurethan, umfassend die Umsetzung einer Polyolkomponente und einer Isocyanatkomponente in Anwesenheit einer wirksamen Menge eines Katalysators und in Anwesenheit einer wirksamen Menge eines Amin/Amids der Struktur I: 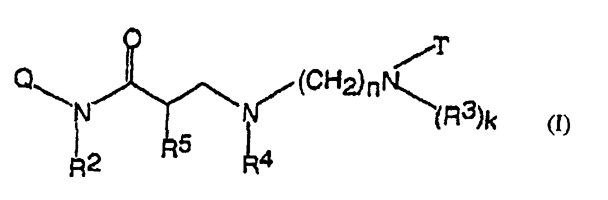 worin
Q C_{z}H_{2z+1} oder (CH₂)ₙN(R³)ₖT bedeutet;
T eine einwertige C₁-C₄-Alkyl-, Amino-C₁-C₄-alkyl-, Mono-C₁-C₄-alklyamino-C₁-C₄-alkyl- oder DiC₁-C₄-alkylamino-C₁-C₄-alkylgruppe ist; oder T eine zweiwertige Alkyl-, Amin-substituierte Alkyl-, Alkylaminoalkyl- oder Alkoxygruppe ist, die mit dem in der Struktur (1) dargestellten Stickstoffatom, an das T gebunden ist, eine Ringstruktur bildet, die sowohl bis zu 6 Ringkohlenstoffatome als auch das in Struktur (1) dargestellte Stickstoffatom umfasst, wobei die Ringstruktur mit C₁- bis C₄-Alkylgruppen substituiert sein kann;
k = 0 oder 1 ist, wobei k = 1 ist, wenn T eine einwertige Gruppe, und k = 0 ist, wenn T eine zweiwertige Gruppe ist;
R² = H oder C_{z}H_{2z+1}; jedes vorhandene R³ = C_{z}H_{2z+1}; R⁴ = H; R⁵ = H oder CH₃; n = 2 bis 6; und jedes vorhandene z = 1 bis 4 ist.

2. Verfahren nach Anspruch 1, wobei n gleich 2 bis 3 und z gleich 1 ist

3. Verfahren nach Anspruch 1, wobei Q = R¹ = C_{z}CH_{2z+1} ist.

4. Verfahren nach einem der Ansprüche 1 bis 3, wobei R¹ Methyl ist.

5. Verfahren nach einem der Ansprüche 1 bis 4, wobei R³ Methyl ist.

6. Verfahren nach einem der Ansprüche 1 bis 5, wobei R⁵ Wasserstoff ist.

7. Verfahren nach einem der Ansprüche 1 bis 6, wobei das Amin/Amid die Struktur 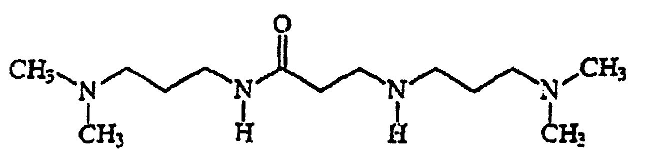 besitzt.

8. Verfahren nach einem der Ansprüche 1 bis 6, wobei T ausgewählt ist aus einer Alkylgruppe mit einem bis vier Kohlenstoffatomen, die Amine tragen oder enthalten, oder einem Alkyl, Amin-substituierten Alkyl, Alkylaminoalkyl oder Alkoxyalkyl, die mit dem in der Formel von Anspruch 1 dargestellten Stickstoffatom, an das T gebunden ist, eine Ringstruktur bildet, die sowohl bis zu 6 Ringkohlenstoffatome als auch das in der Struktur dargestellte Stickstoffatom und gegebenenfalls ein zweites Stickstoffatom oder ein Sauerstoffatom im Ring umfasst, wobei die Ringsstruktur mit C₁- bis C₄-Alkylgruppen substituiert sein kann.

9. Verfahren nach Anspruch 8, wobei T eine Alkylgruppe mit 1 bis 4 Kohlenstoffatomen ist, die einen der mehrer Aminreste tragen kann.

10. Verfahren nach einem der Ansprüche 1 bis 9, wobei das Polyol ein Äquivalentgewicht von 400 bis 1500 und eine mittlere Hydroxylgruppenzahl von 2,1 bis 3,5 aufweist und der Schaumindex zwischen 60 und 130 liegt.

11. Verwendung eines Amin/Amids mit einer Struktur, wie in einem der Ansprüche 1 bis 10 definiert, als Katalysator zur Herstellung eines Polyurethans.

## Revendications

1. Procédé de formation de polyuréthane, comprenant de faire réagir un composant polyol et un composant polyisocyanate en présence d'une quantité efficace d'un catalyseur et d'une quantité efficace d'un amide/amine de structure : 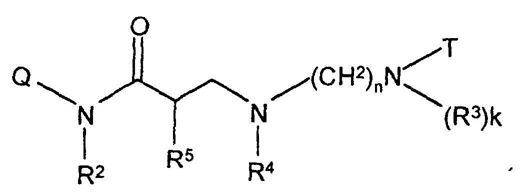 dans laquelle
Q est C_{z}H_{2z+1}, ou (CH₂)ₙN<R³)ₖT ;
T est un groupe monovalent alkyle en C₁-C₄, amino-C₁-C₄-alkyle, mono-C₁-C₄-alkylamino-C₁-C₄-alkyle ou di-C₁-C₄-alkylamino-C₁-C₄-alkyle ou T est un groupe divalent alkyle, alkyle à substitution amino, alkylaminoalkyle ou alcoxyalkyle qui forme avec l'atome d'azote, représenté sur la structure (I) et auquel T est lié, une structure cyclique qui incorpore jusqu'à 6 atomes de carbone dans le cycle ainsi que l'atome d'azote représenté sur la structure (I), ladite structure cyclique pouvant être substituée par un groupe alkyle en C₁ à C₄ ;
k = 0 ou 1, et est égal à 1 si T est un groupe monovalent et à 0 si T est un groupe divalent ;
R² = H ou C_{z}H_{2z+1} ; chaque occurrence de R³ = C_{z}H_{2z+1} ; R⁴ = H ; R⁵ = H ou CH₃ ; n = 2 à 6 ; et chaque occurrence de z = 1 à 4.

2. Procédé selon la revendication 1, dans lequel n est 2 à 3 et z est 1.

3. Procédé selon la revendication 1, dans lequel Q = R¹ = C_{z}H_{2z+1}

4. Procédé selon l'une quelconque des revendications 1 à 3, dans lequel R¹ est un groupe méthyle.

5. Procédé selon l'une quelconque des revendications 1 à 4, dans lequel R³ est un groupe méthyle.

6. Procédé selon l'une quelconque des revendications 1 à 5, dans lequel R⁵ est l'hydrogène.

7. Procédé selon l'une quelconque des revendications 1 à 6, dans lequel 1'amine/amide est de structure :

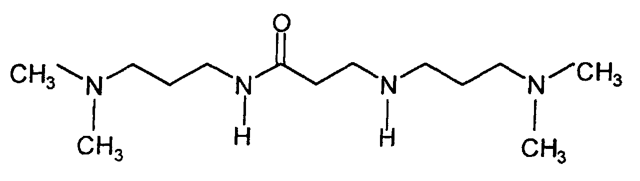

8. Procédé selon l'une quelconque des revendications 1 à 6, dans lequel T est sélectionné dans le groupe comprenant un groupe alkyle de un à quatre atomes de carbone pouvant comporter des amines en surface ou à l'intérieur, ou un groupe alkyle, alkyle à substitution amino, alkylaminoalkyle ou alcoxyalkyle qui forme avec l'atome d'azote, représenté sur la formule de la revendication 1 et auquel T est lié, une structure cyclique qui incorpore jusqu'à 6 atomes de carbone dans le cycle ainsi que l'atome d'azote représenté sur ladite formule et, éventuellement, un deuxième atome d'azote ou un atome d'oxygène dans le cycle, ladite structure cyclique pouvant comprendre des substitutions alkyle en C₁ à C₄ sur le cycle.

9. Procédé selon la revendication 8, dans lequel T est un groupe alkyle de un à quatre atomes de carbone pouvant comporter une ou plusieurs amines en surface.

10. Procédé selon l'une quelconque des revendications 1 à 9, dans lequel le polyol possède un poids équivalent compris entre 400 et 1500 et un nombre moyen de groupes hydroxyle de 2,1 à 3,5, et l'indice de la mousse est compris entre 60 et 130.

11. Utilisation d'un amide/amine, dont la structure est telle que définie dans l'une quelconque des revendications 1 à 10, comme catalyseur pour préparer un polyuréthane.
